Europäisches Patentamt

European Patent Office

Office européen des brevets

(19)

(11) **EP 1 507 217 A1**

(12) **EUROPEAN PATENT APPLICATION**
published in accordance with Art. 158(3) EPC

(43) Date of publication:
**16.02.2005 Bulletin 2005/07**

(51) Int Cl.7: **G06F 17/30**

(21) Application number: **02728114.6**

(22) Date of filing: **22.05.2002**

(86) International application number:
**PCT/JP2002/004961**

(87) International publication number:
**WO 2003/098471 (27.11.2003 Gazette 2003/48)**

(84) Designated Contracting States:
**DE FR GB**

(71) Applicant: **FUJITSU LIMITED**
**Kawasaki-shi, Kanagawa 211-8588 (JP)**

(72) Inventors:
• **SUGIYAMA, Hajime, c/o FUJITSU LIMITED**
**Kawasaki-shi, Kanagawa 211-8588 (JP)**
• **KAWANISHI, Yuichi, c/o FUJITSU LIMITED**
**Kawasaki-shi, Kanagawa211-8588 (JP)**
• **KONDO, Yuji, c/o FUJITSU KYUSHU SYSTEM**
**Fukuoka-shi, Fukuoka 814--8589 (JP)**

• **MASUMOTO, Shigekazu,**
**c/o FUJITSU KYUSHU SYSTEM**
**Sawara-ku, Fukuoka-shi, Fukuoka 814-8589 (JP)**
• **IKEO, Kazuho**
**Mishima-shi, Shizuoka 411-0023 (JP)**

(74) Representative: **Sunderland, James Harry**
**Haseltine Lake,**
**Imperial House,**
**15-19 Kingsway**
**London WC2B 6UD (GB)**

(54) **METHOD OF ANALYZING GENOME**

(57)      Pieces of character information indicating $0^{th}$ to $n^{th}$ bases from a top of a first base sequence (111) are successively deleted from the first base sequence (111), and first partial sequences (113) that includes (n+1) pieces of partial sequences are created. Pieces of character information indicating $0^{th}$ to $m^{th}$ bases from a top of a second base sequence (112) are successively deleted from the second base sequence (112), and (m+1) pieces of second partial sequences (114) are created. The partial sequences in which the pieces of character information prefix-matched completely or partially with pieces of character information indicating the bases in each of the second partial sequences (114) created are arranged are searched in the first partial sequences (113) by, for example, a binary search method. Matched sequence information (115) that includes information on the partial sequences searched in the first partial sequences (113), information on the partial sequences in the second partial sequences, and information on the number of the pieces of prefix-matched character information is extracted. Furthermore, a matrix display image is created based on the match information extracted, and the matrix display image created is displayed.

FIG.1

**Description**

TECHNICAL FIELD

The present invention relates to a method for analyzing a genome, a computer program for analyzing a genome, an apparatus for analyzing a genome, and a terminal or analyzing a genome that compare base sequences for a genetic site prediction or a genome structure analysis.

BACKGROUND ART

Genetic information on a living organism is coded and stored in an arrangement of base sequences of a chromosome in a cell of the living organism. This arrangement of the base sequences is called "genome sequence". It is noted, however, the genetic information is not included in the whole genome sequence, and the genome sequence includes a site that includes the genetic information and a site that does not include the genetic information. The former site is referred to as a "genetic site" that controls the genetic information.

At present, there are following three methods proposed for a genetic site prediction using a computer to predict which site in a genome sequence acts as a gene.

(1) A gene discovery method by comparison of the genome sequence with an open reading frame sequence

In this method, an arrangement of open reading frame sequences in an experimentally obtained site is compared with that of base sequences in a genome sequence. If the arrangements compared are similar to each other, it is predicted that a gene sequence, which corresponds to the open reading frame sequence, is present in the site.

(2) A gene discovery method by a statistical scheme

In this is a method, an arrangement of sequences in a known genetic site is modeled using, for example, a hidden Markov Model. A genetic site is predicted by determining whether an arrangement of base sequences corresponds to the model.

(3) A gene discovering method by comparison of genome sequences

In this is a method, it is determined that a genome site similar in the arrangement of sequences among closely related species is a genetic site, assuming that the genome site has been preserved in evolution.

Conventionally, the methods (1) and (2) are mainly used. However, the method (3) is increasingly expected to realize a higher accuracy in gene discovery.

Various software programs (for example, Harr plot and Dotter) that realize the method (3) are present. However, such computer programs is made in consideration of permitting non-matching of a certain ratio in comparison target sites matched in arrangement of the genome sequence. Due to this, a processing rate is relatively low and large quantities of calculator resources including a memory are used. A size of each possible comparison target genome is about 1 mega base pair (Mbp) (one million bases) at most, so that the conventional software programs are disadvantageously unsuitable for comparison of such genome sites of a size of over 100 Mbp. Furthermore, a calculation time is disadvantageously as long as 30 days or more if the Dotter is used for comparison of genome sites of a size of 5 Mbp.

Furthermore, the genome site includes a part in which sequences identical in arrangement pattern repeatedly appear. Such sequence is referred to as "repeat sequence". The repeat sequence is used as a marker that indicates a position on the genome sequence or indicates signs of an evolution during a genome structure analysis. Thus, various analyses are performed using the repeat sequences.

Conventionally, a repeat sequence having a relatively short pattern (for example, in units of a few bases) is detected using software program for a repeat sequence detection (for example, Repeat Masker or repmask), and a repeat sequence having a relatively long pattern is detected using software program for a matrix display such as the Harr plot. However, if a size of comparison target genomes is large, it disadvantageously takes considerable time for the detection.

The present invention has been achieved to solve the conventional disadvantages. It is an object of the present invention to provide a method for analyzing a genome, a computer program for analyzing a genome, an apparatus for analyzing a genome, and a terminal or analyzing a genome that can perform a genetic site prediction or a genome structure analysis swiftly and efficiently.

DISCLOSURE OF THE INVENTION

To solve the conventional disadvantages and achieve the object, a method for analyzing a genome, a genome analyzing program, a genome analyzing apparatus, and a genome analyzing terminal are constituted so that, when first genome sequence information and second genome sequence information each including base sequences, in which pieces of character information (A, T, G, and C) that indicate four bases of adenine (A), thymine (T), guanine (G), and cytosine (C) are arranged, are input, 0 to $n^{th}$ pieces of character information indicating bases, where n is a positive integer, from the top of the base sequence (hereinafter, "a first base sequence") in the input first genome sequence information are successively deleted and first partial sequences composed by (n+1) partial sequences (hereinafter, "first partial sequences") are created, and 0 to $m^{th}$ pieces of character information indicating bases, where m is a positive integer, from the top of the base sequence (hereinafter, "second base sequence") in the input second genome sequence information are successively deleted and second partial sequences composed by (m+1) partial sequences (hereinafter, "second partial sequence") are created, the partial sequences in the first partial sequences in which the pieces of character information prefix-matched completely or partially to pieces of character information indicating the bases of the respective partial sequences in the second partial sequences constructed at the partial-sequence creating step are arranged are searched by, for example, a binary search method, match information composed by information on the searched partial sequences in the first partial sequences, information on the partial sequences in the second partial sequences, and information on the number of the pieces of prefix-matched character information is extracted, a matrix display image is created based on the extracted match information, and the created matrix display image is displayed.

Further, the partial sequences in the constructed first partial sequences may be rearranged in a predetermined order (an alphabetical order of the pieces of character information indicating the bases of the respective partial sequences in the first partial sequences), and the rearranged partial sequences in the first partial sequences may be searched.

If pieces of overlapped match information are included in the match information, any one of the pieces of overlapped match information may be left and remaining pieces of match information may not be extracted.

The matrix display image may be created based on at least one of cDNA positional information and existing annotation information.

According to these inventions, the match information on the comparison target genome sequences for the genetic site prediction or the genome structure analysis can be efficiently acquired, and displayed so as to facilitate recognizing the information.

BRIEF DESCRIPTION OF THE DRAWINGS

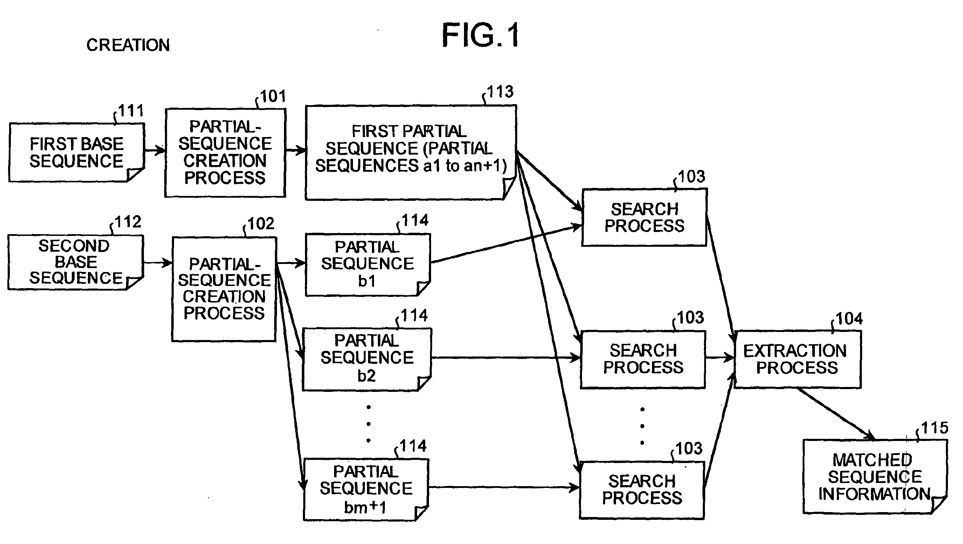

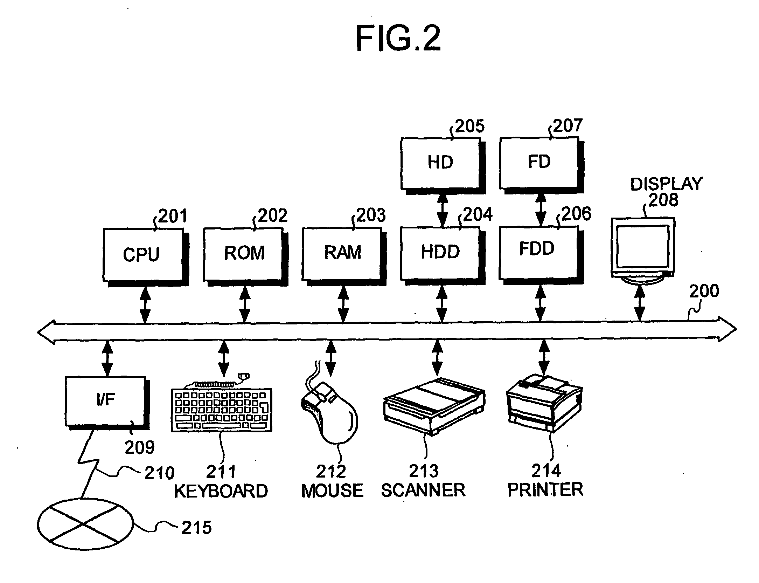

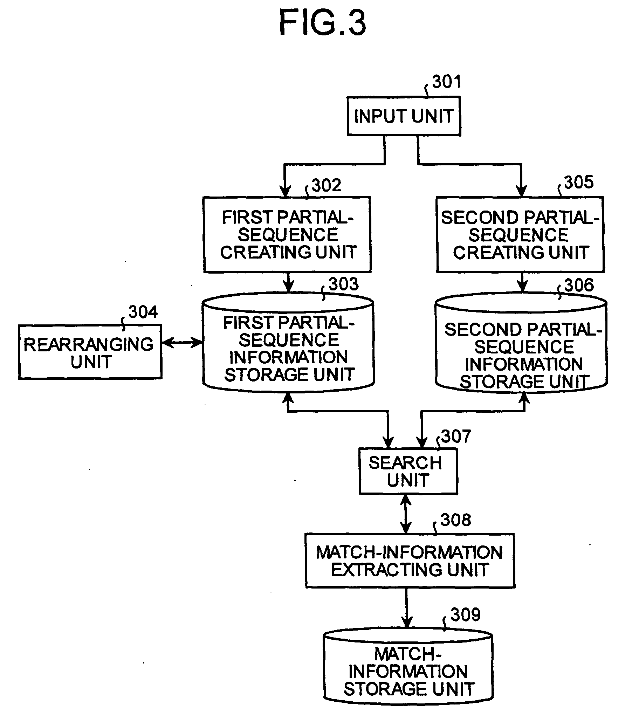

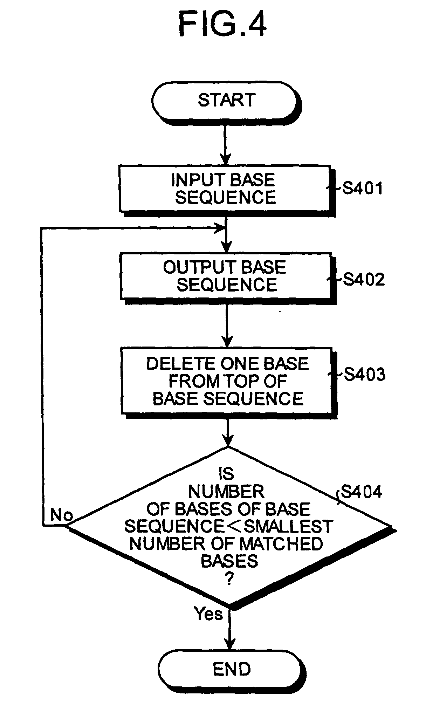

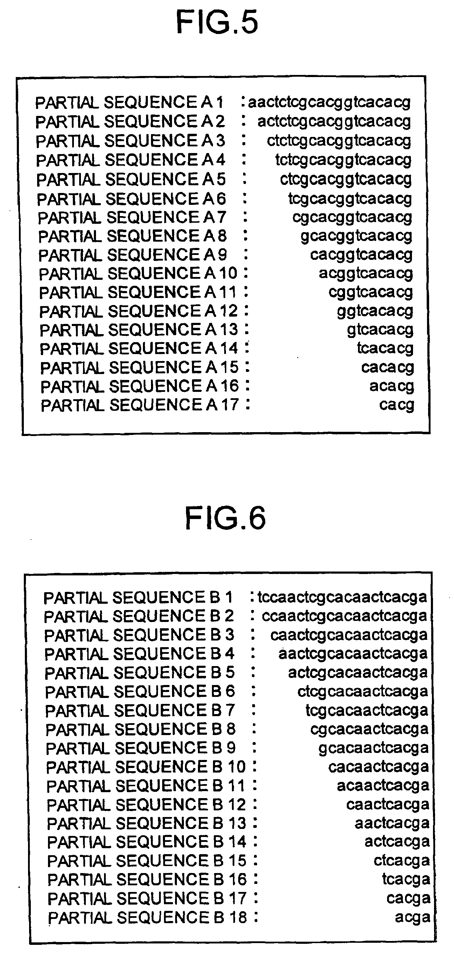

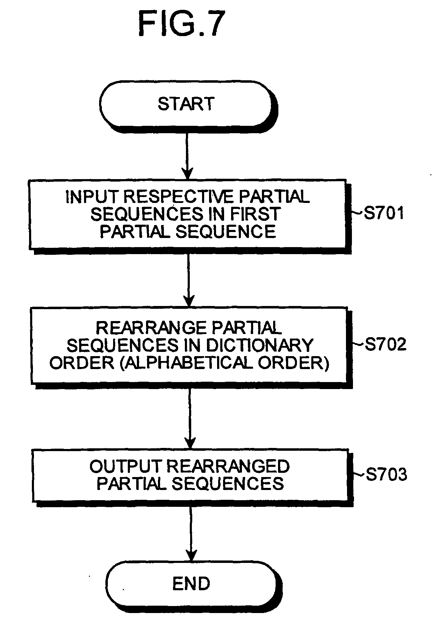

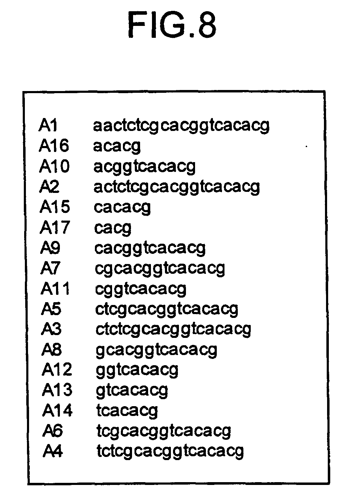

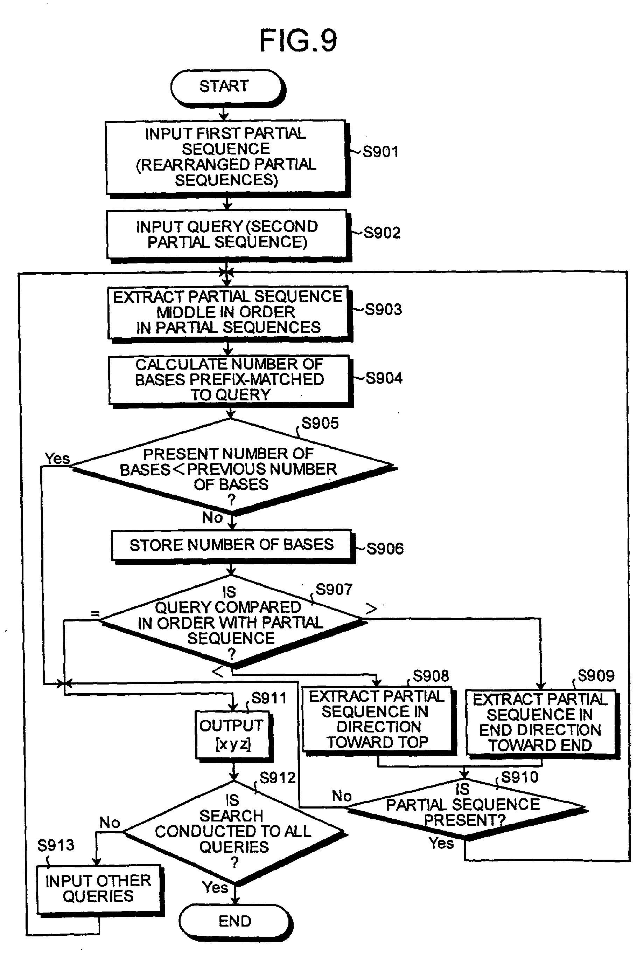

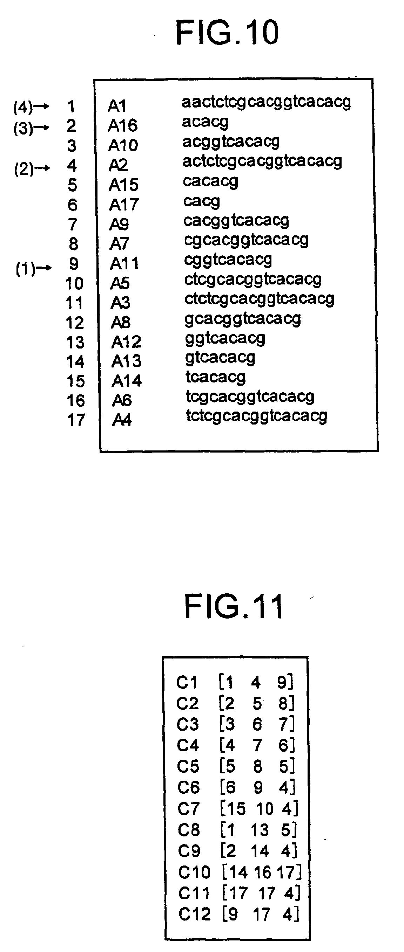

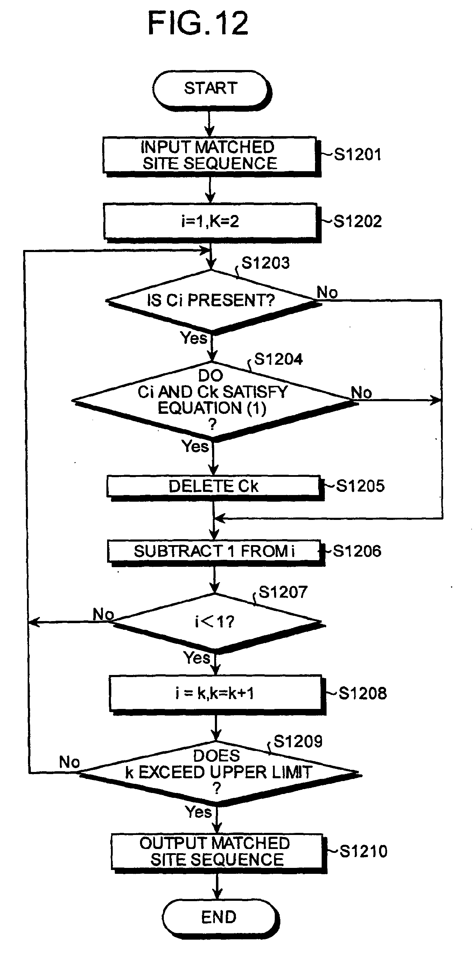

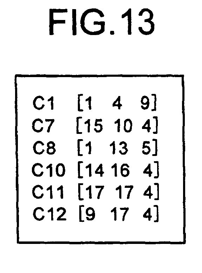

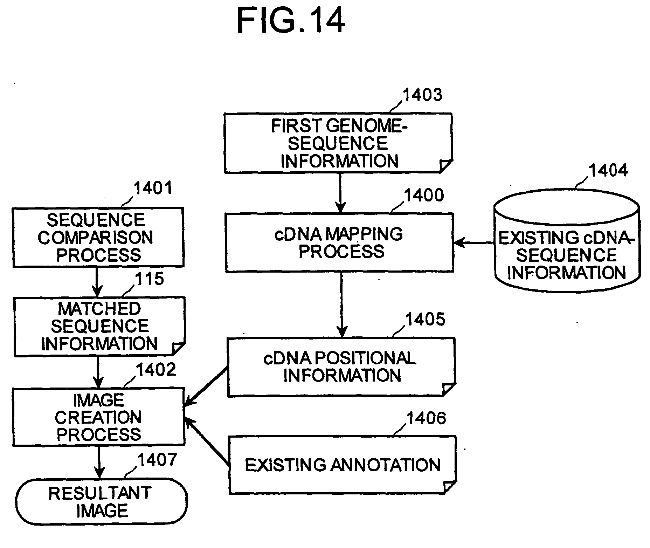

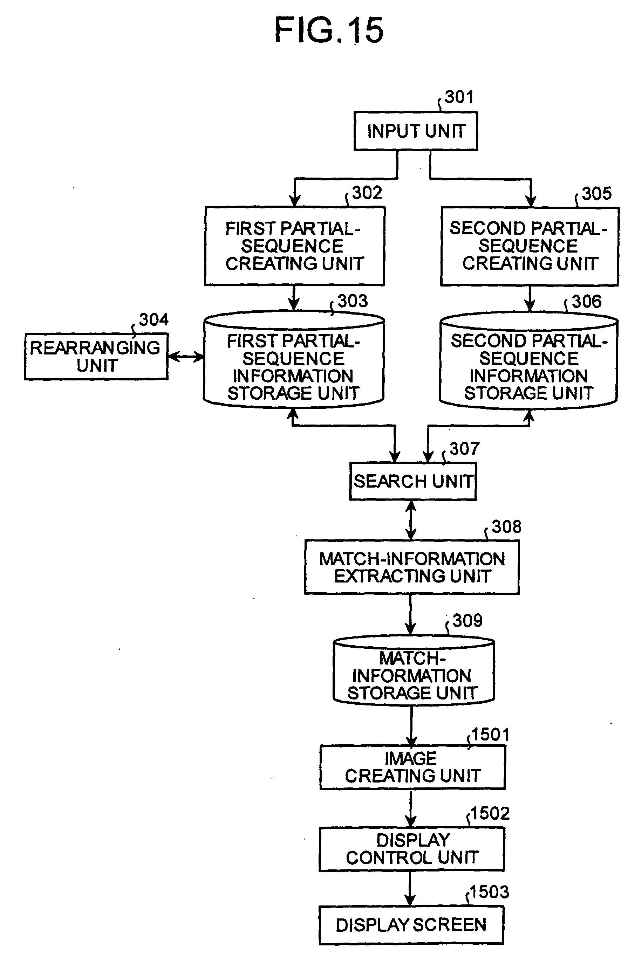

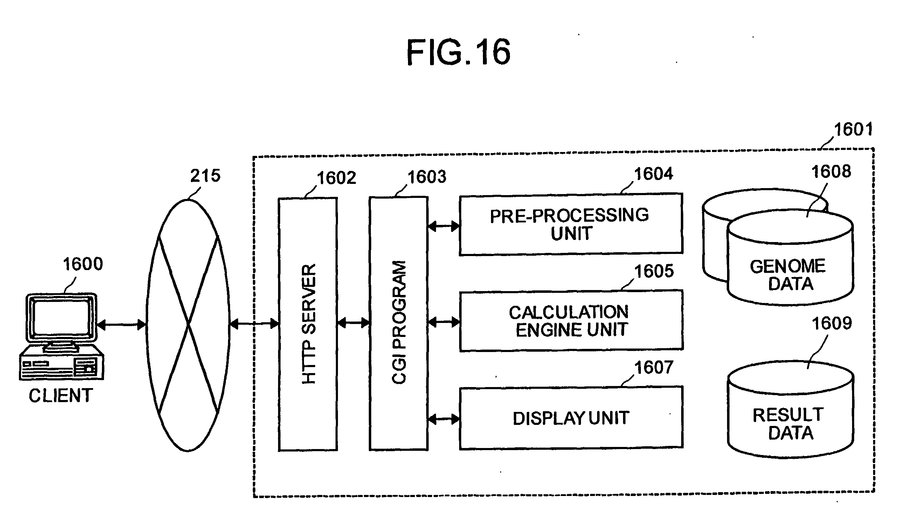

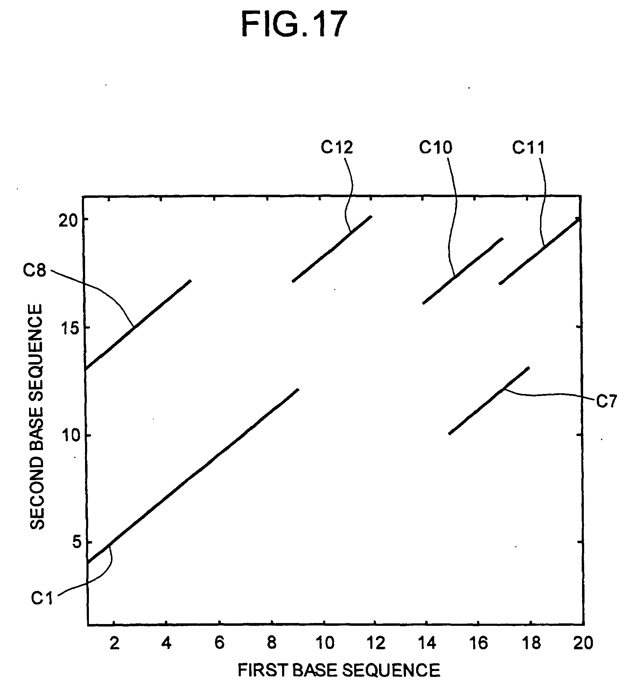

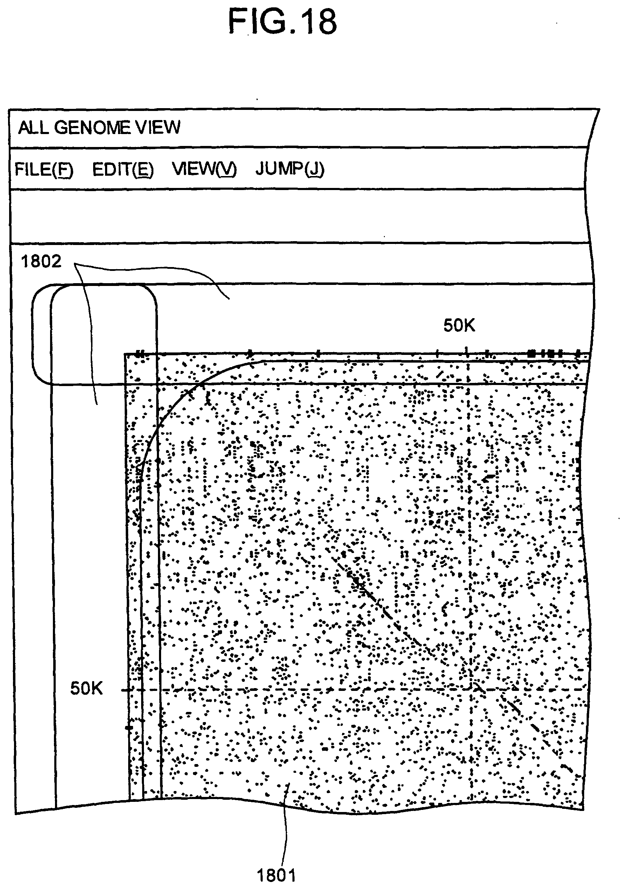

Fig. 1 is an explanatory view for an outline of a sequence comparison process in a genome analysis according to an embodiment of the present invention; Fig. 2 is a block diagram of one example of a hardware configuration of a genome analyzing apparatus according to the embodiment; Fig. 3 is a block diagram of one example of a functional configuration of the genome analyzing apparatus according to the embodiment; Fig. 4 is a flowchart of procedures (for development to partial sequences) performed by the genome analyzing apparatus according to the embodiment; Fig. 5 is an explanatory view for one example of contents of a first partial sequence; Fig. 6 is an explanatory view for one example of contents of a second partial sequence; Fig. 7 is a flowchart of another procedure (for creation of base sequences in a dictionary order) performed by the genome analyzing apparatus according to the embodiment; Fig. 8 is an explanatory view for one example of contents of a rearranged partial sequence; Fig. 9 is a flowchart of still another procedure (for a search and match information extraction) performed by the genome analyzing apparatus according to the embodiment; Fig. 10 is an explanatory view for a binary search method; Fig. 11 is an explanatory view for one example of contents of match information (a matched site sequence); Fig. 12 is a flowchart of still another procedure (for deletion of duplications) performed by the genome analyzing apparatus according to the embodiment; Fig. 13 is an explanatory view for one example of contents of a matched sequence from which the duplications are deleted; Fig. 14 is an explanatory view for an outline of an image creation process and processes around the image creation process performed by the genome analyzing apparatus according to the embodiment; Fig. 15 is a block diagram of another example of the functional configuration of the genome analyzing apparatus according to the embodiment; Fig. 16 is an explanatory view for a system configuration of an entire system including the genome analyzing apparatus; Fig. 17 is an explanatory view for one example of a matrix display image based on the match information; and Fig. 18 is an explanatory view for a part of contents displayed on a matrix display screen.

BEST MODE FOR CARRYING OUT THE INVENTION

Exemplary embodiments of a method for analyzing a genome, a genome analyzing program, and a genome analyzing apparatus according to the present invention will be explained below in detail with reference to the accompanying drawings.

An outline of a sequence comparison process in a genome analysis according to an embodiment of the present invention will be explained first. Fig. 1 is an explanatory view for the outline of the sequence comparison process in the genome analysis according to the embodiment. As shown in Fig. 1, the genome analysis according to the embodiment includes partial-sequence creation processes 101 and 102, a search process 103, and an extraction process 104.

If two pieces of base sequences (a first base sequence 111 and a second base sequence 112) are compared, a first partial sequence (a partial sequence a1 to an+1) 113 is first created from the first base sequence 111 by the partial-sequence creation process 101. A second partial sequence (partial sequences b1, b2, ... and bm+1) 114 is then created from the second base sequence 112 by the partial-sequence creation process 102. Each partial sequence is obtained by deleting an arbitrary number of characters from the top.

A character string search is performed on the first partial sequence 113 using each partial sequence 114 as a key by the search process 103. The extraction process 104 is performed to extract a search result, and removes duplication in the search result to obtain matched sequence information 115.

A hardware configuration of a genome analyzing apparatus according to the embodiment will be explained next. Fig. 2 is a block diagram of one example of the hardware configuration of the genome analyzing apparatus according to the embodiment.

AS shown in Fig. 2, the genome analyzing apparatus includes a central processing unit (CPU) 201, a read-only memory (ROM) 202, a random access memory (RAM) 203, a hard disk drive (HDD) 204, a hard disk (HD) 205, a flexible disk drive (FDD) 206, a flexible disk (FD) 207 as an example of a detachable recording medium, a display 208, an interface (I/F) 209, a keyboard 211, a mouse 212, a scanner 213, and a printer 214. Each of components is connected to one another through a bus 200.

The CPU 201 controls the entire genome analyzing apparatus. The ROM 202 stores programs such as a boot program. The RAM 203 is used as a work area for the CPU 201. The HDD 204 controls read/write of data from/to the HD 205 under control of the CPU 201. The HD 205 stores the data written by controlling the HDD 204.

The FDD 206 controls read/write of data from/ to the FD 207 under control of the CPU 201. The FD 207 stores the data written by controlling the FDD 206 or allows the data stored in the FD 207 to be read by an information processor. Examples of the detachable recording medium may include a compact disc-read-only memory (CD-ROM) (or a compact disc-recordable (CD-R) or a compact disc-rewritable (CD-RW)), a magneto optic disc (MO), a digital versatile disk (DVD), and a memory card as well as the FD 207. The display 208 displays data such as a document, an image, and function information as well as cursors, icons, and tool boxes. The display 208 is, for example, a CRT, a TFT liquid crystal display, or a plasma display.

The I/F 209 is connected to a network 215 such as a LAN or the Internet through a communication line 210, and also connected to another server or the information processor through the network 215. The I/F 209 interfaces the network 215 with an internal unit, and controls input/output of data from/to the other server or the information processor. The I/F 209 is, for example, a modem.

The keyboard 211 includes keys for inputting characters, numbers, various instructions, and the like, and inputs data. The keyboard 211 may be a touch panel input pad or a numeric key pad. The mouse 212 changes a position of a cursor, makes a range selection, moves a window, and changes a window size. As long as equivalent functions as that of the mouse 212 are included, other pointing device such as a track ball or a joystick may be used.

The scanner 213 optically reads image information such as a document, and captures information read into an image processor as image data. The scanner also includes an OCR function that enables the scanner 213 to read printed genome sequence information as data. The printer 214 prints data such as matched sequence information 115. The printer 214 is, for example, a laser printer or an inkjet printer.

A functional configuration of the genome analyzing apparatus will be explained next. Fig. 3 is a block diagram of one example of the functional configuration of the genome analyzing apparatus according to the embodiment. As shown in Fig. 3, the genome analyzing apparatus includes an input unit 301, a first partial-sequence creating unit 302, a first partial-sequence information storage unit 303 a rearranging unit 304, a second partial-sequence creating unit 305, a second partial-sequence information storage unit 306, a search unit 307, a match-information extracting unit 308, and a match-information storage unit 309.

The input unit 301 inputs first genome-sequence information including information on the first base sequence 111 and information on the second base sequence 112 each composed of a base sequence in which character information (A, T, G, and C) that indicate four bases of adenine (A), thymine (T), guanine (G), and a cytosine (C) are arranged.

Specifically, a function of the input unit 301 is realized by, for example, causing the I/F 209 to receive the first and the second genome-sequence information from the network 215. In addition, the function of the input unit 301 may be realized by the FD 207 that is one example of the detachable recording medium, in which the first and the second genome-sequence information are stored, and the FDD 206. Further, the function may be realized by the scanner 213 that includes the OCR function, the keyboard 211, and the mouse 212.

The first partial-sequence creating unit 302 controls the partial-sequence creation process 101 shown in Fig. 1. Namely, the first partial-sequence creating unit 302 successively deletes character information indicating $0^{th}$ to $n^{th}$ (where n is a positive integer) bases from the top of the first base sequence 111 that is the base sequence of the first genome-sequence information input by the input unit 301. In addition, the first partial-sequence creating unit 302 creates a first partial sequence 113 that is a partial sequence including (n+1) pieces of partial sequences. While in the embodiment, the character information is successively deleted from the top, the character information may be successively deleted from the end conversely.

The first partial-sequence information storage unit 303 stores information on the first partial sequence 113 created by the first partial-sequence creating unit 302. Alternatively, the first partial-sequence information storage unit 303 may store the information on the first partial sequence 113 that is created by another apparatus or the like in advance. A function of the first partial-sequence information storage unit 303 is realized by the ROM 202, the RAM 203, the HD 205 and the HDD 204, or the FD 207 and the FD 206.

The rearranging unit 304 rearranges the partial sequences in the information on the first partial sequence 113 created by the first partial-sequence creating unit 302 and stored in the first partial-sequence information storage unit 303 in a predetermined order. The predetermined order may be, for example, a dictionary order of pieces of character information that indicate bases of the respective partial sequences in the first partial sequences 113, that is, an alphabetical order. In the rearrangement in the dictionary order (alphabetical order), if top bases are composed of a same character, a character that appears next in the partial sequence is compared. By repeating this comparison, orders of all partial sequences are determined. For example, if the partial sequences "aa", "ac", "aaa", and "aaaa" are compared, the partial sequences are rearranged in an order of (1) "aa", (2) "aaa", (3) "aaaa", and (4) ac.

The second partial-sequence creating unit 305 successively deletes character information indicating $0^{th}$ to $m^{th}$ (where m is a positive integer) bases from the top of the second base sequence 112 that is the base sequence of the second genome-sequence information input. In addition, the second partial-sequence creating unit 305 constructs second partial sequences 114 that are partial sequences composed of (m+1) pieces of partial sequences. While in the embodiment, the character data is successively deleted from the top, the character information may be successively deleted conversely from the end.

The second partial-sequence information storage unit 306 stores information on the second partial sequences 114 created by the second partial-sequence creating unit 305. Alternatively, the second partial-sequence information storage unit 306 may store the information on the second partial sequences 114 constructed by another apparatus or the like in advance. A function of the second partial-sequence information storage unit 306 is realized by the ROM 202, the RAM 203, the HD 205 and the HDD 204, or the FD 207 and the FD 206.

The search unit 307 searches for partial sequences in the first partial sequences, which are created by the first partial-sequence creating unit 305 or stored in the first partial-sequence information storage unit 303, and in which pieces of character information prefix-matched completely or partially to the pieces of character information indicating the bases of information on the respective second partial sequences created by the second partial-sequence creating unit 305 or stored in the second partial-sequence information storage unit 306 are arranged. The partial sequences in the first partial sequences stored in the first partial-sequence information storage unit 303 may be rearranged by the rearranging unit 304.

The search unit 307 may search for partial sequences in the first partial sequences 113 in which pieces of character information prefix-matched completely or partially to the pieces of character information indicating the bases of the respective second partial sequences created by the partial-sequence creating unit are arranged, by a binary search method. The binary search method will be explained later.

The search unit 307 may search for a partial sequence having a largest number of pieces of the character information that prefix-matches completely or partially to the pieces of character information indicating the bases of the respective partial sequences 114 created by the partial-sequence creating unit among those in the first partial sequences 113 in which the pieces of character information prefix-matched completely or partially to the pieces of character information indicating the bases of the respective partial sequences 114 are arranged.

The match-information extracting unit 308 extracts match information (matched site sequences) that includes information on the partial sequences in the first partial sequence searched by the search unit 307, information on the partial sequences 114, and information on the number of pieces of the character information prefix-matched. Further, it is preferable that if there are duplicate pieces of the match information in the match information extracted, the match-information extracting unit 308 leaves any one of the duplicate pieces of the match information, and does not extract the other duplicate pieces of match information.

Functions of the first partial-sequence creating unit 302, the rearranging unit 304, the second partial-sequence creating unit 305, the search unit 307, and the match-information extracting unit 308 are realized by making the CPU 201 execute a program stored in the ROM 202, the RAM 203, the HD 205, or the FD 207.

Furthermore, the match-information storage unit 309 stores the match information extracted by the match-information extracting unit 308 in a state in which the information can be used. A function of the match-information storage unit 309 is realized by the ROM 202, the RAM 203, the HD 205 and the HDD 204, or the FD 207 and the FDD 206.

A process procedure performed by the genome analyzing apparatus will be explained next. The process includes (1) a process for developing a base sequence A to partial sequences, (2) a process for developing a base sequence B to partial sequences, (3) a process for creating base sequences in a dictionary-order, (4) a process for searching and extracting the match information, and (5) a process for deleting duplication. The processes (1) to (3) may be performed in advance as pre-processes.

It is assumed herein that base sequences to be a comparison target are the following two pieces of sequences. The following explanation applies even when the base sequences A and B are replaced. The base sequences to be the comparison target may be equal in length.

> Base sequence A: aactctcgcacggtcacacg (20 bases)
> Base sequence B: tccaactcgcacaactcacga (21 bases)

If it is detected that the two pieces of the base sequences of the comparison target are matched in an arrangement, it is assumed that they are matched in a direction of arrangement, that is, they are prefix-matched. To detect that the two pieces of the base sequences of the comparison target are matched in an opposite direction of the arrangement to the former direction, one of the base sequences may be arranged in an opposite direction.

(1) Development of base sequence A to partial sequences

One of the base sequences to be the comparison target (the base sequence A in the embodiment) is developed to partial sequences by deleting one base from the top of the base sequence A. Each of the partial sequences is denoted by Ai. The development of the base sequence A to the partial sequences is performed until the number of characters of a last partial sequence is the smallest number of matched bases (a smallest length of a part matched in arrangement of bases detected by comparing arrangements). For example, the smallest number of matched bases is four in this embodiment.

Fig. 4 is a flowchart of the process (for development to the partial sequences) performed by the genome analyzing apparatus according to the embodiment. As shown in the flowchart in Fig. 4, the base sequence A (aactctcgcacggtcacacg (20 bases)) serving as the first base sequence 111 is input (read) (step S401). The base sequence A (aactctcgcacggtcacacg (20 bases)) is output (step S402). This base sequence output is a partial sequence A1.

One base (a) positioned at the top of the partial sequence A1 output at the step S402 is deleted (step S403). Therefore, the partial sequence A1 is developed to a sequence (actctcgcacggtcacacg (19 bases)), which is a partial sequence A2. It is determined whether the number of bases of the base sequence is smaller than the smallest number of matched sequences (step S404). If it is determined that the number of bases of the base sequence is larger than or equal to the smallest number of matched sequences ("NO" at step S404), the process returns to the step S402, at which the partial sequence A2 (actctcgcacggtcacacg (19 bases)) is output. Furthermore, one base (a) positioned at the top of this partial sequence A2 is deleted (step S403), thus, the partial sequence A2 is developed to a sequence (ctctcgcacggtcacacg (18 bases)), which is a partial sequence A3.

The steps S402 to S404 are repeatedly executed. If it is determined that the number of bases of the base sequence is smaller than the smallest number of matched bases at the step S404 ("YES" at step S404), the process is finished. Since the smallest number of matched bases is set at "4", if the number of bases is "4" as a result of deletion at the step S403, the partial base sequence is output (at the step S402), and if the number of bases is "3", the processing is finished. In Fig. 5, 17 pieces of the partial sequences (the first partial sequences 113) A1 to A17 created through the above steps are shown.

(2) Development of base sequence B to partial sequences

With similar procedures, partial sequences are created from the base sequence B (tccaactcgcacaactcacga (21 bases)). In Fig. 6, 18 pieces of partial sequences B1 to B18 created similarly through the above steps are shown.

(3) Creation of base sequences in a dictionary-order

The partial sequences Ai are rearranged in the dictionary order. It is assumed herein that a set of the partial sequences thus arranged herein is a rearranged partial sequence set {Ai}. Fig. 7 is a flowchart of the other process (for constructing the dictionary-order base sequence set). As shown in the flowchart of Fig. 7, the respective partial sequences Ai in the first partial sequence 113 are input (read) (step S701).

The partial sequences Ai input are rearranged in the dictionary order (alphabetical order), in other words, the partial sequences Ai are sorted (step S702). The rearrangement is made in the dictionary order (alphabetical order). Namely, the partial sequences Ai are rearranged in an order of (1) A (adenine), (2) T (thymine), (3) G (guanine), and (4) C (cytosine). Bases positioned at the top in the partial sequences are compared so as to rearrange the partial sequences in the predetermined order, irrespective of lengths of the base sequences. If the bases at the top in the partial sequences are same, bases that appear next are compared to rearrange the partial sequences in the predetermined order. By repeating this comparison, all partial sequences are arranged in the predetermined order.

Thereafter, the rearranged partial sequences {Ai} are output (step S703), and the process is finished. In Fig. 8, the rearranged partial sequences {Ai} are shown.

(4) Search and extraction of match information

The binary search method is applied to the partial sequences {Ai} in the dictionary order to search the partial sequence Ai that includes bases that prefix-matches to the partial sequences Bi (where i=1 to 17) for equal to or more than the smallest number (four bases in the following case) of matched bases. Fig. 9 is a flowchart of the other process (for searching and extracting the match information) performed by the genome analyzing apparatus according to the embodiment.

As shown in the flowchart in Fig. 9, the first partial sequence, which is the rearranged partial sequence {A1} is input (step S901). Then, a query, which is a second partial sequence By is input (step S902). An example of inputting "B4 (aactcgcacaactcacga)" as the query will be explained herein. A partial sequence Ai (1) that is middle in the order in the rearranged partial sequences {A1} is extracted (step S903). As shown in Fig. 10, the middle partial sequence is a ninth partial sequence in the order "A11 (cggtcacacg)" since, for example, the total number (17) of the rearranged partial sequences {A1}÷2=8.5.

The query By is compared with the partial sequence Ai (1), and the number of prefix-matched bases is calculated (step S904). A number of bases obtained at a present comparison is compared with a number of bases obtained at a previous comparison. If the number of bases obtained at the present comparison is equal to or larger than the number of bases obtained at the previous comparison ("NO" at step S905), the number of bases of the present comparison is stored in a predetermined storage region (step S906). If the number of bases of the previous comparison is larger than the number of bases of the present comparison ("YES" at step S905), the process goes to a step S911 without executing anything. Namely, at the step S911, the number of bases of the previous comparison is set as the match information.

When the query B4 (aactcgcacaactcacga) is compared with the partial sequence A11 (cggtcacacg), the number of bases prefix-matched is "0". Since information on the number of bases obtained at the previous comparison is not present in the comparison of the query B4 with the partial sequence A11, this number of bases "0" is stored.

The query By is compared in order with the partial sequence Ai (1) (step S907). If they are completely matched to each other (By=Ai (1) at step S907), this indicates that the partial sequence to be searched is discovered. Therefore, nothing is performed thereafter, and the process goes to the step S911.

If the comparison of the query By with the partial sequence Ai (1) in order indicates that the query By is higher in order than the partial sequence A (1) (By<Ai (1) at step S907), the partial sequence to be searched can be judged to be located in direction toward the top. Therefore, a partial sequence located in the direction toward the top relative to the partial sequence Ai (1) is extracted (step S908).

If the comparison of the query By with the partial sequence Ai (1) in order indicates that the query By is lower in order than the partial sequence A (1) (By>Ai (1) at the step S907), the partial sequence to be searched can be judged to be located in a direction toward the end. Therefore, a partial sequence located in the direction toward the end relative to the partial sequence Ai (1) is extracted (at a step S909).

It is determined whether the partial sequence is present in either the direction toward the top or the end (at a step S910). If the partial sequence is present ("YES" at step S910), the processing returns to the step S903. If no partial sequence is present ("NO" at step S910), this indicates that no further matched sequence is present and the process proceeds to the step S911.

If the query B4 (aactcgcacaactcacga) is compared in order with the partial sequence A11 (cggtcacacg), bases located at the top the query B4 and the partial sequence A11 are "a" and "c", respectively. In addition, the query B4 is higher in order than the partial sequence A11, the process goes to the step S908, at which eight pieces of partial sequences (A1, A16, A10, A2, A15, A17, A9, and A7) located in the direction toward the top are extracted. The process then returns to the step S903.

At the step S903, the partial sequence middle in the order among the eight pieces of the partial sequences is extracted. Specifically, since the total number (8)÷2=4, "the partial sequence A2 (actctcgcacggtcacacg)" that is the fourth from the top is extracted. If the query B4 (aactcgcacaactcacga) is then compared with the partial sequence A2 (actctcgcacggtcacacg), the number of prefix-matched bases "a" is "1". Since the information on the number of bases of the previous comparison of the query B4 with the partial sequence A11 is "0", the number of bases "1" is stored.

If the query B4 (aactcgcacaactcacga) is compared in order with the partial sequence A2 (actctcgcacggtcacacg), bases located a the top of the query B4 and the partial sequence A2 are both "a" and second bases are "a" and "c", respectively. In addition, the query B4 is higher in order than the partial sequence A2. Therefore, the process goes to the step S908 at which three pieces of the partial sequences (A1, A16, and A10) in the direction toward the top are extracted. The process then returns to the step S903.

At the step S903, the partial sequence middle in the order among the three pieces of the partial sequences is extracted. Specifically, since the total number (3)÷2=1.5, the "partial sequence A16 (acacg)" that is the second from the top is extracted. If the query B4 (aactcgcacaactcacga) is then compared with the partial sequence A16 (acacg), the number of bases prefix-matched "a" is "1". Since the information on the number of bases of the previous comparison of the query B4 with the partial sequence A11 is "1", the number of bases "1" is stored.

If the query B4 (aactcgcacaactcacga) is compared in order with the partial sequence A16 (acacg), bases located at the top of the query B4 and the partial sequence A16 are both "a" and second bases are "a" and "c" respectively. In addition, the query B4 is higher in order than the partial sequence A16. Therefore, the process goes to the step S908 at which one partial sequence (A1) in the direction toward the top is extracted. The process then returns to the step S903.

At the step S903, the partial sequence middle in the order among the one piece of partial sequence is extracted. Since only one piece of the partial sequence is present, the "partial sequence A1 (aactctcgcacggtcacacg)" is extracted. If the query B4 (aactcgcacaactcacga) is then compared with the partial sequence A1 (aactctcgcacggtcacacg), the number of prefix-matched bases "a" is "9". Since the information on the number of bases of the previous comparison of the query B4 with the partial sequence A1 is "1", the number of bases "9" is stored.

If the query B4 (aactcgcacaactcacga) is compared in order with the partial sequence A1 (aactctcgcacggtcacacg), ninth bases from the top are same and tenth bases of the query B4 and the partial sequence A1 are "g" and "c" respectively. In addition, the query B4 is higher in order than the partial sequence A16. Therefore, the process goes to the step S908 at which one piece of partial sequence (A1) in the direction toward the top is extracted. However, since no partial sequence is left ("NO" at step S910), the comparison is not repeated any longer and the process goes to a step S911.

At the step S911, the largest value among those stored at the step S906 is set at "z" and an index of the partial sequence Ai at the value z is set at "x", an index of the query By at the value z is set at "y", and a set of three numbers [x y z] is output (step S911). If a plurality of largest values is present, sets of three numbers [x y z] are respectively output. As for a query B4, the number of bases is "9" and the partial sequence is A1. Therefore, a set of three numbers is [1 4 9]. This means that the arrangement of nine bases from the top in the base sequence A is matched to that of nine bases from the fourth base in the base sequence B.

It is then determined whether the search is conducted to all the queries (step S912). If the search is not conducted to all the queries ("NO" at step S912), remaining other queries are input (step S913) and the process returns to the step S903. If the search is conducted to all the queries ("YES" at step S912), the process is finished. If the process is simply repeated from B1 to B17, data shown in Fig 11 is obtained. This data is match information (matched site sequences {Ci}).

(5) Deletion of duplication

Among the match site sequences {Ci}, C2 means that the arrangement of eight bases from the second in the base sequence A is matched to that of eight sequences from the fifth in the base sequence B. C1 means that the arrangement of nine bases from the first in the base sequence A is matched to that of nine bases from the fourth in the base sequence B. Accordingly, the matched sequence C2 is included in the matched sequence C1. To delete such duplication, a following process is performed. It is noted that this process can be performed while performing the search and extraction of the match information.

Ci [ai bi ni] is compared with Ck [ak bk nk]. If they satisfy a relationship of the following equation (1), it is defined that the matched sequence Ci includes the matched sequence Ck and the sequence Ck is deleted. It is noted, however, that the sequences Ci and Ck that satisfy i<k are selected.

$$ak-ai=bk-bi=ni-nk \qquad (1)$$

Fig. 12 is a flowchart of the other process (for deleting duplication) performed by the genome analyzing apparatus according to the embodiment. As shown in the flowchart in Fig. 12, the matched site sequences Ci and Ck are input (read) (step S1201). 1 is substituted to the sequence Ci and 2 is substituted to the sequence Ck, whereby specifying the comparison target sequences C1 and C2 (step S1202).

It is determined whether the sequence Ci is present, in other words, whether the Ci is deleted at a previous step S1205 (step S1203). If the Ci is deleted and not present ("NO" at step S1203), nothing is performed and the process proceeds to a step S1206. If the Ci is not deleted and is present ("YES" at step S1203), the process goes to a step S1204. As for the C1 and C2 initially specified, since the C1 is not deleted (the C1 is not to be deleted), the process goes to the step S1204.

At the step S1204, it is determined whether the Ci and Ck satisfy the equation (1). If they do not satisfy the equation (1) ("NO" at step S1204), nothing is performed and the process proceeds to a step S1206. If they satisfy the equation (1) ("YES" at step S1204), the Ck is deleted from the matched site sequences (at step S1205). If the sequences C1 [1 4 9] and C2 [2 5 8] are applied to the equation (1), then ak-ai=1, bk-bi=1, and ni-nk=1, and a relationship of ak-ai=bk-bi=ni-nk is established. Accordingly, the C2 is deleted from the matched site sequences.

After 1 is subtracted from i (step S1206), it is determined whether the resultant i is smaller than 1 (step S1207). If the i is not smaller than 1 ("NO" at step S1207), the process returns to the step S1203 and the steps S1203 to S1207 are repeated. If it is determined at the step S1207 that the i is smaller than 1, which means the i is 0 ("YES" at step S1207), a value k is substituted to the i and 1 is added to the k (step S1208).

It is determined whether the value k to which 1 is added at the step S1208 exceeds an upper limit, in other words, whether the total number of matched site sequences input at the step S1201 (step S1209). If the k does not exceed the upper limit ("NO" at step S1209), the process returns to the step S1203 and the steps S1203 to S1209 are repeated. If it is determined at the step S1209 that the k exceeds the upper limit ("YES" at step S1209), the matched site sequences that have not been deleted but remained are output (at step S1210) and the process is finished.

As for the C1, since 1 is subtracted from i, a result is 0. Therefore, at the step S1208, the C1 is replaced by C2 and the C2 is replaced by C3. Since the C3 does not exceed the upper limit, the process returns to the step S1203. However, since the C2 is already deleted and not present, the C2 is further changed to the C1 and the process returns again to the step S1203. Since the C1 is present this time, it is determined whether C1 [149] and C3 [3 6 7] satisfy the relationship of the equation (1). If the C1 [1 4 9] and the C3 [3 6 7] are applied to the equation (1), ak-ai=2, bk-bi=2, and ni-nk=2 and a relationship of ak-ai=bk-bi=ni-nk is established. Accordingly, the C3 is also deleted from the matched site sequences.

The same process is then repeated. It is determined whether C1 and C4, C1 and C5, C1 and C6, C1 and C7, C7 and C8, C1 and C8, C8 and C9, C8 and C10, C7 and C10, C1 and C10, C10 and C11, C8 and C11, C7 and C11, C1 and C11, C11 and C12, C10 and C12, C8 and C12, C7 and C12, and C1 and C12 satisfy the relationship of the equation (1) respectively in this order. As a result of the determination, the C1 and C4, the C1 and C5, the C1 and C6, and the C8 and C9 satisfy the equation (1). Accordingly, the C4, C5, C6 and C9 are deleted from the matched site sequences. Details of the determination are as follows.

[1] Compare the C1 with the C2. If i=1 and k=2, the C1 and C2 satisfy the equation (1) and the C1 includes the C2. The C2 is, therefore, deleted.

[2] Compare the C1 with the C3. If i=1 and k=3, the C1 and C3 satisfy the equation (1) and the C1 includes the C3. The C3 is, therefore, deleted.

[3] Compare the C1 with the C4. If i=1 and k=4, the C1 and C4 satisfy the equation (1) and the C1 includes the C4. The C4 is, therefore, deleted.

[4] Compare the C1 with the C5. If i=1 and k=5, the C1 and C5 satisfy the equation (1) and the C1 includes the C5. The C5 is, therefore, deleted.

[5] Compare the C1 with the C6. If i=1 and k=6, the C1 and C6 satisfy the equation (1) and the C1 includes the C6. The C6 is, therefore, deleted.

[6] Compare the C1 with the C7. If i=1 and k=7, the C1 and C7 do not satisfy the equation (1). The C7 is, therefore, not deleted.

[7] Compare the C7 with the C8. If i=7 and k=8, the C7 and C8 do not satisfy the equation (1). The C8 is, therefore, not deleted.

[8] Compare the C1 with the C8. If i=1 and k=8, the C7 and C8 do not satisfy the equation (1). The C8 is, therefore, not deleted.

[9] Compare the C8 with the C9. If i=8 and k=9, the C1 and C8 satisfy the equation (1) and the C8 includes the C9. The C9 is, therefore, deleted.

[10] Compare the C8 with the C10. If i=8 and k=10, the C8 and C10 do not satisfy the equation (1). The C10 is, therefore, not deleted.

[11] Compare the C7 with the C10. If i=7 and k=10, the C7 and C10 do not satisfy the equation (1). The C10 is, therefore, not deleted.

[12] Compare the C1 with the C10. If i=1 and k=10, the C1 and C10 do not satisfy the equation (1). The C10 is, therefore, not deleted.

[13] Compare the C10 with the C11. If i=10 and k=11, the C10 and C11 do not satisfy the equation (1). The C11 is, therefore, not deleted.

[14] Compare the C8 with the C11. If i=8 and k=11, the C8 and C11 do not satisfy the equation (1). The C11 is, therefore, not deleted.

[15] Compare the C7 with the C11. If i=7 and k=11, the C7 and C11 do not satisfy the equation (1). The C11 is, therefore, not deleted.

[16] Compare the C1 with the C11. If i=1 and k=11, the C1 and C11 do not satisfy the equation (1). The C11 is, therefore, not deleted.

[17] Compare the C11 with the C12. If i=11 and k=12, the C11 and C12 do not satisfy the equation (1). The C12 is, therefore, not deleted.

[18] Compare the C10 with the C12. If i=10 and k=12, the C10 and C12 do not satisfy the equation (1). The C12 is, therefore, not deleted.

[19] Compare the C9 with the C12. If i=9 and k=12, the C9 and C12 do not satisfy the equation (1). The C12 is, therefore, not deleted.

[20] Compare the C8 with the C12. If i=8 and k=12, the C8 and C12 do not satisfy the equation (1). The C12 is, therefore, not deleted.

[21] Compare the C7 with the C12. If i=7 and k=12, the C7 and C12 do not satisfy the equation (1). The C12 is, therefore, not deleted.

[21] Compare the C1 with the C12. If i=1 and k=12, the C1 and C12 do not satisfy the equation (1). The C12 is, therefore, not deleted.

As a consequence, the match information (matched site sequence) from which duplication is deleted is shown in Fig. 13. The match information thus obtained is stored in the match-information storage unit 309 to be used in the genetic site prediction or the genome structure analysis.

An outline of an image creation process and processes around the image creation using the matched sequence information 115 obtained by the sequence comparison process explained above will be explained next. Fig. 14 is an explanatory view for the outline of the image creation process and processes around the image creation process performed by the genome analyzing apparatus according to the embodiment.

As shown in Fig. 14, the matched sequence information 115 extracted by a sequence comparison process 1401 (see Fig. 1) is used in an image creation process 1402. Furthermore, a cDNA mapping process 1400 is performed based on first genome-sequence information 1403 and existing cDNA-sequence information 1404, thereby obtaining cDNA positional information 1405.

In the image creation process 1402, the matched sequence information 115 is displayed in a matrix. In addition, the cDNA positional information 1405 and existing annotation information 1406 are superimposed with an image displayed as the matrix, thereby creating a resultant image 1407. Using the resultant image 1407, the matched sequence information 115, the cDNA positional information 1405, and the existing annotation information 1406 can be checked at a same time on one display screen.

The cDNA mapping processing 1400 is a process for comparing a target genome sequence (the first genome-sequence information 1403) with the existing cDNA sequence (the existing cDNA sequence 1404), and calculating cDNA positional information as to "which site on the genome sequence is homologous to which existing cDNA". Since the sequence comparison process 1401 is already explained above, the explained is omitted.

(Functional configuration of the genome analyzing apparatus)

The functional configuration of the genome analyzing apparatus for the image creation process 1402 will be explained. Fig. 15 is a block diagram of another example of the functional configuration of the genome analyzing apparatus according to the embodiment. As shown in Fig. 15, the genome analyzing apparatus includes not only the input unit 301, the first partial-sequence creating unit 302, the first partial-sequence information storage unit 303, the rearranging unit 304, the second partial-sequence creating unit 305, the second partial-sequence information storage unit 306, the search unit 307, the match-information extracting unit 308, and the match-information storage unit 309 that are explained with reference to Fig. 3 but also an image creating unit 1501, a display control unit 1502, and a display screen 1503. Each of components of the input unit 301, the first partial-sequence creating unit 302, the first partial-sequence information storage unit 303, the rearranging unit 304, the second partial-sequence creating unit 305, the second partial-sequence information storage unit 306, the search unit 307, the match-information extracting unit 308, and the match-information storage unit 309 are same as those explained with reference to Fig. 3. Therefore, detailed explanations of such components are omitted.

The image creating unit 1501 creates a matrix display image based on the match information extracted by the match-information extracting unit 308. The image creating unit 1401 may create the matrix display image based on at least one of the cDNA positional information 1405 and the existing annotation information 1406.

More specifically, the image creating unit 1501 may create the matrix display image as a graph that indicates a length that corresponds to the number of the character information prefix-matched where the information on the partial sequences in the first partial sequence among the match information extracted by the match information extracting unit 308 and the information on the partial sequence in the second partial sequence among the match information extracted by the match information extracting unit 308 are used for either of a vertical axis and a horizontal axis respectively. Further, the image creating unit 1501 may make at least one of the cDNA positional information and the existing annotation information correspond to at least one of the vertical axis and the horizontal axis.

The display control unit 1502 controls the display screen 1503 to display the matrix display image created by the image creating unit 1501. A function of each of the image creating unit 1501 and the display control unit 1502 is realized by making the CPU 201 execute a program stored in the ROM 202, the RAM 203, the HD 205, or the FD 207.

The matrix display image is displayed on the display screen 1503. Specifically, a function of the display screen 1503 is realized by, for example, the display 208 shown in Fig. 2.

A system configuration of an entire system that includes the genome analyzing apparatus according to the embodiment will be explained. Fig. 16 is an explanatory view for the system configuration of the entire system that includes the genome analyzing apparatus according to the embodiment. As shown in Fig. 16, reference symbol 1601 denotes the genome analyzing apparatus that is connected to a client (terminal) 1600 through the network 215 such as the Intranet. The genome analyzing apparatus 1601 includes a hyper-text-transfer-protocol (HTTP) server 1602 that controls a network communications with the client 1600 using a communication protocol for transmitting and receiving a hyper-text-markup-language (HTML) document between a world wide web (WWW) server and a WWW client, a common-gateway-interface (CGI) program 1603 that is an interface for calling a desired external program and transmitting a program execution result to the WWW browser in response to a request from the WWW browser, a pre-processing unit 1604 that performs pre-processes such as the partial-sequence creation processes 101 and 102 in the cDNA mapping process 1400 and the cDNA mapping process 1401, a calculation engine unit 1605 that performs the search process 103 and the extraction process 104 in the sequence comparison process 1401, the image creation process 1402, and the like, a display unit 1607 that displays the resultant image 1407 and the like, and a result database 1609 that stores process results.

Such a system configuration enables a user to also view the resultant image 1407 created by, for example, data input and operation input from the client 1600 on the display screen of the client 1600.

A content of the matrix display image created using the match information (matched site sequence) shown in Fig. 13 will be explained. Fig. 17 is an explanatory view for one example of the matrix display image based on the match information. As shown in Fig. 17, the image creation process 1402 draws a graph having positions of axes corresponding to those on base sequences based on the matched site sequences or the matched site sequences from which the duplication are deleted. Specifically, the horizontal axis (x) indicates the first base sequence and the vertical axis (y) indicates the second base sequence.

If so, the sequence C1 [1 4 9] is drawn as a graph having (x, y) continuing in an upper right direction from a coordinate (1, 4) by "9" that is the number of the bases matched. The same is true for the C7, C8, C10, C11, and C12. By thus displaying, a degree of matching between the two pieces of base sequences can be efficiently recognized.

Contents of the matrix display screen that include the cDNA positional information 1405 and the existing annotation information 1406 superimposed on the matrix display images will be explained. Fig. 18 is an explanatory view for a part displayed contents on the matrix display screen. As shown in Fig. 18, a reference symbol 1801 denotes a display region of the matrix display image, a reference symbol 1802 denotes a display region of each of the cDNA positional information 1405 and the existing annotation information 1406. As shown in Fig. 18, by superimposing the DNA positional information 1405 and the existing annotation information 1406 on the matrix display image, the matched sequence information 115, the cDNA positional information 1405, and the existing annotation information 1406 can be checked at a same time on one display screen. It is thereby possible to efficiently perform the genetic site prediction based on mutual relationship among them.

As explained above, according to the embodiment, a comparison over a whole genome size of 100 Mbp (several hundred million bases) can be performed, and the match information on the genome sequences to be the comparison target for the genetic site prediction or the genome structure analysis can be efficiently acquired. Specifically, to perform a 5-Mbp comparison, it takes about one month if the Dotter is used. The genome analyzing apparatus according to the embodiment, by contrast, can perform calculation within about one hour. Furthermore, the match information acquired can be displayed so as to facilitate recognition of the match information.

Moreover, the method for analyzing a genome according to the embodiment may be a computer-readable program prepared in advance, and may be realized by making a computer, such as a personal computer or a workstation, execute the program. This program is recorded in a computer-readable recording medium, such as an HD, an FD, a CD-ROM, an MO, or a DVD, and executed by being read from the recording medium by the computer. This program may be a transmission medium that can be distributed through a network such as the Internet.

As explained above, according to the present invention, the match information of the genome sequences that are the comparison target for the genetic site prediction or the genome structure analysis can be efficiently acquired, and displayed so as to facilitate recognition of the information. Thus, it is possible to obtain the method for analyzing a genome, the genome analyzing program, the genome analyzing apparatus, and the genome analyzing terminal capable of performing the genetic site prediction or the genome structure analysis swiftly and efficiently.

INDUSTRIAL APPLICABILITY

As explained above, the method for analyzing a genome, the genome analyzing program, the genome analyzing apparatus, and the genome analyzing terminal according to the present invention are useful for the genetic site prediction or the genome structure analysis. Particularly, it is possible to facilitate the base sequence comparison for the genetic site prediction or the genome structure analysis, therefore, he method for analyzing a genome, the genome analyzing program, the genome analyzing apparatus, and the genome analyzing terminal are suitable for performing the genetic site prediction or the genome structure analysis swiftly and efficiently.

**Claims**

1. A method for analyzing a genome comprising:

inputting first genome-sequence information and second genome-sequence information, the first genome-sequence information and the second genome-sequence information including base sequences that indicates four bases of adenine, thymine, guanine, and cytosine arranged in the base sequences;
creating a partial sequence that includes

creating a first partial sequence by successively deleting $0^{th}$ to $n^{th}$ pieces of character information that indicates bases, where n is a positive integer, from a top of the base sequences in the first genome-sequence information such that the first partial sequence composed of (n+1) pieces of partial sequences; and
creating a second partial sequence by successively deleting $0^{th}$ to $m^{th}$ pieces of the character information that indicates bases, where m is a positive integer, from a top of the base sequences in the second genome-sequence information such that the second partial sequences composed of (m+1) pieces of partial sequences;

searching, in the first partial sequence, the partial sequence that prefix-matches completely or partially with pieces of character information, which indicates the bases of the respective partial sequence in the second partial sequence created at the creating the partial sequence, are arranged;
extracting match information that includes information on the partial sequence in the first partial sequence searched at the searching, information on the partial sequence in the second partial sequence, and information on a number of the pieces of prefix-matched character information;
creating a matrix display image based on the match information extracted at the extracting; and
displaying the matrix display image created at the creating the matrix display image.

2. The method according to claim 1, further comprising rearranging the partial sequence in the first partial sequence creating the first partial sequence in a predetermined order, wherein
the searching includes searching the partial sequence in the first partial sequence rearranged at the rearranging.

3. The method according to claim 2, wherein the predetermined order is an alphabetical order of the character information that indicates the bases of each of the partial sequences in the first partial sequence.

4. The method according to claim 2 or 3, wherein the searching includes searching, in the first partial sequence, the partial sequence that prefix-matches completely or partially with the pieces of the character information, which indicates the bases of the respective partial sequence in the second partial sequence created at the creating the partial sequence, by binary search.

5. The method according to any one of claims 1 to 3, wherein
the searching includes searching a partial sequence having a largest number of pieces of character information from among the partial sequences in the first partial sequence that prefix-matches completely or partially with the pieces of the character information that indicates the bases of the respective partial sequence in the second partial sequence created at the creating the partial sequence.

6. A method for analyzing a genome comprising:

inputting first partial sequence created by successively deleting $0^{th}$ to $n^{th}$ pieces of character information that indicates bases, where n is a positive integer, from a top of the base sequences in first genome-sequence information such that the first partial sequence is composed of (n+1) partial sequences, the first genome-sequence information including base sequences, in which pieces of character information that indicate four bases of adenine, thymine, guanine, and cytosine are arranged, and second partial sequence that is created by successively deleting $0^{th}$ to $m^{th}$ pieces of the character information that indicate bases, where m is a positive integer, from a top of the base sequences in second genome-sequence information such that the second partial sequence is composed of (m+1) pieces of partial sequences, the second genome-sequence information including base sequences, in which pieces of the character information that indicate the four bases of adenine, thymine, guanine, and cytosine are arranged;
searching, in the first partial sequence, a partial sequence that prefix-matches completely or partially with pieces of character information that indicates bases of a partial sequence in the second partial sequence input;
extracting match information that includes information on the partial sequence in the first partial sequence searched at the searching, information on the partial sequence in the second partial sequence, and information on a number of the pieces of prefix-matched character information;
creating a matrix display image based on the match information extracted at the extracting the matched information; and
displaying the matrix display image created at the creating the matrix display image.

7. The method according to any one of claims 1, 2, 3 and 6, wherein if there are duplicate pieces of the match information among the match information, the extracting includes leaving any one of the duplicate pieces of the match information without extracting other duplicate pieces of the match information.

8. The method according to any one of claims 1, 2, 3, and 6, wherein the creating the matrix display image includes creating the matrix display image based on at least one of information on a position of cDNA and information on existing annotation.

**9.** The method according to claim 8, wherein the creating the matrix display image includes creating the matrix display image as a graph that indicates a length corresponding to the number of the pieces of the prefix-matched character information, with information on the partial sequences in the first partial sequence and information on the partial sequences in the second partial sequence among the match information extracted at the extracting set as a vertical axis and a horizontal axis or the horizontal axis and the vertical axis, respectively, and causes at least one of the information on the position of cDNA and the information on the existing annotation to correspond to at least one of the vertical axis and the horizontal axis.

**10.** A computer program for analyzing a genome, the computer program making a computer execute:

inputting first genome-sequence information and second genome-sequence information, the first genome-sequence information and the second genome-sequence information including base sequences that indicates four bases of adenine, thymine, guanine, and cytosine arranged in the base sequences; creating a partial sequence that includes

creating a first partial sequence by successively deleting $0^{th}$ to $n^{th}$ pieces of character information that indicates bases, where n is a positive integer, from a top of the base sequences in the first genome-sequence information such that the first partial sequence composed of (n+1) pieces of partial sequences; and creating a second partial sequence by successively deleting $0^{th}$ to $m^{th}$ pieces of the character information that indicates bases, where m is a positive integer, from a top of the base sequences in the second genome-sequence information such that the second partial sequences composed of (m+1) pieces of partial sequences;

searching, in the first partial sequence, the partial sequence that prefix-matches completely or partially with pieces of character information, which indicates the bases of the respective partial sequence in the second partial sequence created at the creating the partial sequence, are arranged; extracting match information that includes information on the partial sequence in the first partial sequence searched at the searching, information on the partial sequence in the second partial sequence, and information on a number of the pieces of prefix-matched character information;

creating a matrix display image based on the match information. extracted at the extracting; and displaying the matrix display image created at the creating the matrix display image.

**11.** The computer program according to claim 10, further making a computer execute rearranging the partial sequence in the first partial sequence creating the first partial sequence in a predetermined order, wherein

the searching includes searching the partial sequence in the first partial sequence rearranged at the rearranging.

**12.** The computer program according to claim 11, wherein the searching includes searching, in the first partial sequence, the partial sequence that prefix-matches completely or partially with the pieces of the character information, which indicates the bases of the respective partial sequence in the second partial sequence created at the creating the partial sequence, by binary search.

**13.** The computer program according to any one of claims 10 to 12, wherein

the searching includes searching a partial sequence having a largest number of pieces of character information from among the partial sequences in the first partial sequence that prefix-matches completely or partially with the pieces of the character information that indicates the bases of the respective partial sequence in the second partial sequence created at the creating the partial sequence.

**14.** A computer program for analyzing a genome, the computer program making a computer execute:

inputting first partial sequence created by successively deleting $0^{th}$ to $n^{th}$ pieces of character information that indicates bases, where n is a positive integer, from a top of the base sequences in first genome-sequence information such that the first partial sequence is composed of (n+1) partial sequences, the first genome-sequence information including base sequences, in which pieces of character information that indicate four bases of adenine, thymine, guanine, and cytosine are arranged, and second partial sequence that is created by successively deleting $0^{th}$ to $m^{th}$ pieces of the character information that indicate bases, where m is a positive integer, from a top of the base sequences in second genome-sequence information such that the second partial sequence is composed of (m+1) pieces of partial sequences, the second genome-sequence information including base sequences, in which pieces of the character information that indicate the four bases of adenine, thymine, guanine, and cytosine are arranged;

searching, in the first partial sequence, a partial sequence that prefix-matches completely or partially with pieces of character information that indicates bases of a partial sequence in the second partial sequence input;

extracting match information that includes information on the partial sequence in the first partial sequence searched at the searching, information on the partial sequence in the second partial sequence, and information on a number of the pieces of prefix-matched character information;

creating a matrix display image based on the match information extracted at the extracting the matched information; and

displaying the matrix display image created at the creating the matrix display image.

15. The computer program according to any one of claims 10, 11, 12 and 14, wherein if there are duplicate pieces of the match information among the match information, the extracting includes leaving any one of the duplicate pieces of the match information without extracting other duplicate pieces of the match information.

16. The computer program according to claims 10, 11, 12, and 14, wherein the creating the matrix display image includes creating the matrix display image based on at least one of information on a position of cDNA and information on existing annotation.

17. An apparatus for analyzing a genome comprising:

an input unit that accepts input of first genome-sequence information and second genome-sequence information, the first genome-sequence information and the second genome-sequence information including base sequences that indicates four bases of adenine, thymine, guanine, and cytosine arranged in the base sequences;

a creating unit that creates partial sequences that includes

a first partial sequence by successively deleting $0^{th}$ to $n^{th}$ pieces of character information that indicates bases, where n is a positive integer, from a top of the base sequences in the first genome-sequence information such that the first partial sequence composed of (n+1) pieces of partial sequences; and

a second partial sequence by successively deleting $0^{th}$ to $m^{th}$ pieces of the character information that indicates bases, where m is a positive integer, from a top of the base sequences in the second genome-sequence information such that the second partial sequences composed of (m+1) pieces of partial sequences;

a searching unit that searches, in the first partial sequence, the partial sequence that prefix-matches completely or partially with pieces of character information, which indicates the bases of the respective partial sequence in the second partial sequence created at the creating the partial sequence, are arranged;

an extracting unit that extracts match information that includes information on the partial sequence in the first partial sequence searched at the searching, information on the partial sequence in the second partial sequence, and information on a number of the pieces of prefix-matched character information;

an image creating unit that creates a matrix display image based on the match information extracted at the extracting; and

a displaying unit that displays the matrix display image created at the creating the matrix display image.

18. The apparatus according to claim 17, wherein

the searching unit searches a partial sequence having a largest number of pieces of character information from among the partial sequences in the first partial sequence that prefix-matches completely or partially with the pieces of the character information that indicates the bases of the respective partial sequence in the second partial sequence created at the creating the partial sequence.

19. An apparatus for analyzing a genome comprising:

an input unit that accepts input of first partial sequence created by successively deleting $0^{th}$ to $n^{th}$ pieces of character information that indicates bases, where n is a positive integer, from a top of the base sequences in first genome-sequence information such that the first partial sequence is composed of (n+1) partial sequences, the first genome-sequence information including base sequences, in which pieces of character information that indicate four bases of adenine, thymine, guanine, and cytosine are arranged, and second partial sequence that is created by successively deleting $0^{th}$ to $m^{th}$ pieces of the character information that indicate bases, where m is a positive integer, from a top of the base sequences in second genome-sequence information such that the second partial sequence is composed of (m+1) pieces of partial sequences, the second genome-sequence information including base sequences, in which pieces of the character information that indicate the four bases of adenine, thymine, guanine, and cytosine are arranged;

a searching unit that searches, in the first partial sequence, a partial sequence that prefix-matches completely or partially with pieces of character information that indicates bases of a partial sequence in the second partial sequence input;

an extracting unit that extracts match information that includes information on the partial sequence in the first partial sequence searched at the searching, information on the partial sequence in the second partial sequence, and information on a number of the pieces of prefix-matched character information;

an image creating unit that creates a matrix display image based on the match information extracted at the extracting the matched information; and

a displaying unit that displays the matrix display image created at the creating the matrix display image.

20. The apparatus according to any one of claims 17 to 19, wherein if there are duplicate pieces of the match information among the match information, the extracting unit leaves any one of the duplicate pieces of the match information without extracting other duplicate pieces of the match information.

21. The apparatus according to any one of claims 17 to 19, wherein the image creating unit creates the matrix display image based on at least one of information on a position of cDNA and information on existing annotation.

# FIG.1

CREATION

- 111 — FIRST BASE SEQUENCE
- 112 — SECOND BASE SEQUENCE
- 101 — PARTIAL-SEQUENCE CREATION PROCESS
- 102 — PARTIAL-SEQUENCE CREATION PROCESS
- 113 — FIRST PARTIAL SEQUENCE (PARTIAL SEQUENCES a1 to an+1)
- 114 — PARTIAL SEQUENCE b1
- 114 — PARTIAL SEQUENCE b2
- 114 — PARTIAL SEQUENCE bm+1
- 103 — SEARCH PROCESS
- 103 — SEARCH PROCESS
- 103 — SEARCH PROCESS
- 104 — EXTRACTION PROCESS
- 115 — MATCHED SEQUENCE INFORMATION

# FIG.2

EP 1 507 217 A1

# FIG.3

INPUT UNIT 301

FIRST PARTIAL-
SEQUENCE
CREATING UNIT 302

SECOND PARTIAL-
SEQUENCE
CREATING UNIT 305

REARRANGING
UNIT 304

FIRST PARTIAL-
SEQUENCE
INFORMATION
STORAGE UNIT 303

SECOND PARTIAL-
SEQUENCE
INFORMATION
STORAGE UNIT 306

SEARCH
UNIT 307

MATCH-INFORMATION
EXTRACTING UNIT 308

MATCH-
INFORMATION
STORAGE UNIT 309

# FIG.4

START

INPUT BASE SEQUENCE ~S401

OUTPUT BASE SEQUENCE ~S402

DELETE ONE BASE FROM TOP OF BASE SEQUENCE ~S403

IS NUMBER OF BASES OF BASE SEQUENCE < SMALLEST NUMBER OF MATCHED BASES ? ~S404

No

Yes

END

# FIG.5

```
PARTIAL SEQUENCE A 1    : aactctcgcacggtcacacg
PARTIAL SEQUENCE A 2    :  actctcgcacggtcacacg
PARTIAL SEQUENCE A 3    :   ctctcgcacggtcacacg
PARTIAL SEQUENCE A 4    :    tctcgcacggtcacacg
PARTIAL SEQUENCE A 5    :     ctcgcacggtcacacg
PARTIAL SEQUENCE A 6    :      tcgcacggtcacacg
PARTIAL SEQUENCE A 7    :       cgcacggtcacacg
PARTIAL SEQUENCE A 8    :        gcacggtcacacg
PARTIAL SEQUENCE A 9    :         cacggtcacacg
PARTIAL SEQUENCE A 10  :          acggtcacacg
PARTIAL SEQUENCE A 11  :           cggtcacacg
PARTIAL SEQUENCE A 12  :            ggtcacacg
PARTIAL SEQUENCE A 13  :             gtcacacg
PARTIAL SEQUENCE A 14  :              tcacacg
PARTIAL SEQUENCE A 15  :               cacacg
PARTIAL SEQUENCE A 16  :                acacg
PARTIAL SEQUENCE A 17  :                 cacg
```

# FIG.6

```
PARTIAL SEQUENCE B 1    : tccaactcgcacaactcacga
PARTIAL SEQUENCE B 2    :  ccaactcgcacaactcacga
PARTIAL SEQUENCE B 3    :   caactcgcacaactcacga
PARTIAL SEQUENCE B 4    :    aactcgcacaactcacga
PARTIAL SEQUENCE B 5    :     actcgcacaactcacga
PARTIAL SEQUENCE B 6    :      ctcgcacaactcacga
PARTIAL SEQUENCE B 7    :       tcgcacaactcacga
PARTIAL SEQUENCE B 8    :        cgcacaactcacga
PARTIAL SEQUENCE B 9    :         gcacaactcacga
PARTIAL SEQUENCE B 10  :          cacaactcacga
PARTIAL SEQUENCE B 11  :           acaactcacga
PARTIAL SEQUENCE B 12  :            caactcacga
PARTIAL SEQUENCE B 13  :             aactcacga
PARTIAL SEQUENCE B 14  :              actcacga
PARTIAL SEQUENCE B 15  :               ctcacga
PARTIAL SEQUENCE B 16  :                tcacga
PARTIAL SEQUENCE B 17  :                 cacga
PARTIAL SEQUENCE B 18  :                  acga
```

# FIG.7

START

INPUT RESPECTIVE PARTIAL
SEQUENCES IN FIRST
PARTIAL SEQUENCE — S701

REARRANGE PARTIAL
SEQUENCES IN DICTIONARY
ORDER (ALPHABETICAL ORDER) — S702

OUTPUT REARRANGED
PARTIAL SEQUENCES — S703

END

# FIG.8

| | |
|---|---|
| A1 | aactctcgcacggtcacacg |
| A16 | acacg |
| A10 | acggtcacacg |
| A2 | actctcgcacggtcacacg |
| A15 | cacacg |
| A17 | cacg |
| A9 | cacggtcacacg |
| A7 | cgcacggtcacacg |
| A11 | cggtcacacg |
| A5 | ctcgcacggtcacacg |
| A3 | ctctcgcacggtcacacg |
| A8 | gcacggtcacacg |
| A12 | ggtcacacg |
| A13 | gtcacacg |
| A14 | tcacacg |
| A6 | tcgcacggtcacacg |
| A4 | tctcgcacggtcacacg |

FIG.9

START

INPUT FIRST PARTIAL SEQUENCE (REARRANGED PARTIAL SEQUENCES) — S901

INPUT QUERY (SECOND PARTIAL SEQUENCE) — S902

EXTRACT PARTIAL SEQUENCE MIDDLE IN ORDER IN PARTIAL SEQUENCES — S903

CALCULATE NUMBER OF BASES PREFIX-MATCHED TO QUERY — S904

S905
PRESENT NUMBER OF BASES < PREVIOUS NUMBER OF BASES ?
Yes / No

STORE NUMBER OF BASES — S906

S907
IS QUERY COMPARED IN ORDER WITH PARTIAL SEQUENCE ?
= / < / >

S908 EXTRACT PARTIAL SEQUENCE IN DIRECTION TOWARD TOP

S909 EXTRACT PARTIAL SEQUENCE IN END DIRECTION TOWARD END

S911 OUTPUT [x y z]

S910 IS PARTIAL SEQUENCE PRESENT? No / Yes

S912 IS SEARCH CONDUCTED TO ALL QUERIES ? No / Yes

S913 INPUT OTHER QUERIES

END

23

# FIG.10

| | | |
|---|---|---|
| (4)→ 1 | A1 | aactctcgcacggtcacacg |
| (3)→ 2 | A16 | acacg |
| 3 | A10 | acggtcacacg |
| (2)→ 4 | A2 | actctcgcacggtcacacg |
| 5 | A15 | cacacg |
| 6 | A17 | cacg |
| 7 | A9 | cacggtcacacg |
| 8 | A7 | cgcacggtcacacg |
| (1)→ 9 | A11 | cggtcacacg |
| 10 | A5 | ctcgcacggtcacacg |
| 11 | A3 | ctctcgcacggtcacacg |
| 12 | A8 | gcacggtcacacg |
| 13 | A12 | ggtcacacg |
| 14 | A13 | gtcacacg |
| 15 | A14 | tcacacg |
| 16 | A6 | tcgcacggtcacacg |
| 17 | A4 | tctcgcacggtcacacg |

# FIG.11

```
C1  [1   4   9]
C2  [2   5   8]
C3  [3   6   7]
C4  [4   7   6]
C5  [5   8   5]
C6  [6   9   4]
C7  [15 10   4]
C8  [1  13   5]
C9  [2  14   4]
C10 [14 16  17]
C11 [17 17   4]
C12 [9  17   4]
```

# FIG.12

START

↓

INPUT MATCHED SITE SEQUENCE — S1201

↓

$i=1, K=2$ — S1202

↓

S1203
IS $C_i$ PRESENT? — No

Yes ↓

S1204
DO $C_i$ AND $C_k$ SATISFY EQUATION (1) ? — No

Yes ↓

DELETE $C_k$ — S1205

↓

SUBTRACT 1 FROM $i$ — S1206

↓

S1207
$i < 1$? — No

Yes ↓

$i = k, k = k+1$ — S1208

↓

S1209
DOES $k$ EXCEED UPPER LIMIT ? — No

Yes ↓

OUTPUT MATCHED SITE SEQUENCE — S1210

↓

END

# FIG.13

```
C1   [1   4   9]
C7   [15 10  4]
C8   [1   13  5]
C10  [14 16  4]
C11  [17 17  4]
C12  [9   17  4]
```

# FIG.14

FIRST GENOME-SEQUENCE INFORMATION (1403)

SEQUENCE COMPARISON PROCESS (1401)

cDNA MAPPING PROCESS (1400)

EXISTING cDNA-SEQUENCE INFORMATION (1404)

MATCHED SEQUENCE INFORMATION (115)

cDNA POSITIONAL INFORMATION (1405)

IMAGE CREATION PROCESS (1402)

EXISTING ANNOTATION (1406)

RESULTANT IMAGE (1407)

# FIG.15

```
                                    ┌─────────────┐ 301
                                    │ INPUT UNIT  │
                                    └─────────────┘
                        ┌──────────────────┴──────────────────┐
                        ▼ 302                                  ▼ 305
              ┌──────────────────┐              ┌──────────────────┐
              │  FIRST PARTIAL-  │              │ SECOND PARTIAL-  │
              │    SEQUENCE      │              │    SEQUENCE      │
              │  CREATING UNIT   │              │  CREATING UNIT   │
              └──────────────────┘              └──────────────────┘
                        │ 303                              │ 306
                        ▼                                  ▼
   304      ┌──────────────────┐              ┌──────────────────┐
┌──────────┐│  FIRST PARTIAL-  │              │ SECOND PARTIAL-  │
│REARRANGING│◄─►│   SEQUENCE   │              │    SEQUENCE      │
│   UNIT    ││  INFORMATION     │              │  INFORMATION     │
└──────────┘│  STORAGE UNIT    │              │  STORAGE UNIT    │
            └──────────────────┘              └──────────────────┘
                        └────────────┬──────────────┘
                                     ▼ 307
                            ┌──────────────┐
                            │ SEARCH UNIT  │
                            └──────────────┘
                                     │ 308
                                     ▼
                         ┌──────────────────────┐
                         │  MATCH-INFORMATION    │
                         │  EXTRACTING UNIT      │
                         └──────────────────────┘
                                     │ 309
                                     ▼
                            ┌──────────────┐
                            │   MATCH-     │
                            │ INFORMATION  │
                            │ STORAGE UNIT │
                            └──────────────┘
                                     │ 1501
                                     ▼
                            ┌──────────────┐
                            │    IMAGE     │
                            │ CREATING UNIT│
                            └──────────────┘
                                     │ 1502
                                     ▼
                            ┌──────────────┐
                            │   DISPLAY    │
                            │ CONTROL UNIT │
                            └──────────────┘
                                     │ 1503
                                     ▼
                            ┌──────────────┐
                            │DISPLAY SCREEN│
                            └──────────────┘
```

# FIG.16

EP 1 507 217 A1

# FIG.17

# FIG.18

| INTERNATIONAL SEARCH REPORT | International application No. |
|---|---|
| | PCT/JP02/04961 |

**A. CLASSIFICATION OF SUBJECT MATTER**
Int.Cl$^7$ G06F17/30

According to International Patent Classification (IPC) or to both national classification and IPC

**B. FIELDS SEARCHED**

Minimum documentation searched (classification system followed by classification symbols)
Int.Cl$^7$ G06F17/30

Documentation searched other than minimum documentation to the extent that such documents are included in the fields searched

| | | | |
|---|---|---|---|
| Jitsuyo Shinan Koho | 1922–1996 | Toroku Jitsuyo Shinan Koho | 1994–2002 |
| Kokai Jitsuyo Shinan Koho | 1971–2002 | Jitsuyo Shinan Toroku Koho | 1996–2002 |

Electronic data base consulted during the international search (name of data base and, where practicable, search terms used)
JICST FILE(JOIS), WPI, INSPEC(DIALOG)

**C. DOCUMENTS CONSIDERED TO BE RELEVANT**

| Category* | Citation of document, with indication, where appropriate, of the relevant passages | Relevant to claim No. |
|---|---|---|
| A | MIYAKE et al., Kakusan Hairetsu Bunseki heno Fuzzy Matching Kansu no Oyo, 6th Fuzzy System Symposium Koen Ronbunshu, 06 September, 1990 (06.09.90), pages 307 to 310 | 1-21 |
| A | CHAO K-M, MILLER W. Linear-Space Algorithms that Build Local Alignments from Fragments. Algorithmica, Vol.13, No.1/2, 1995, pages 106 to 134 | 1-21 |

☐ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>26 June, 2002 (26.06.02) | Date of mailing of the international search report<br>09 July, 2002 (09.07.02) |
| Name and mailing address of the ISA/<br>Japanese Patent Office | Authorized officer |
| Facsimile No. | Telephone No. |

Form PCT/ISA/210 (second sheet) (July 1998)